# EUROPEAN PATENT SPECIFICATION

(11) **EP 3 328 799 B1**
(45) Date of publication and mention of the grant of the patent: **15.07.2020**
(21) Application number: 16750420.8
(22) Date of filing: 29.07.2016
(51) Int. Cl.: C02F 3/34, C12N 1/20

(54) **CONSORTIUM OF MICRO-ORGANISMS AND ITS USE TO REDUCE CHEMICAL OXYGEN DEMAND OF SPENT METAL WORKING FLUID**
VEREINIGUNG AUS MIKROORGANISMEN UND DEREN VERWENDUNG ZUR REDUZIERUNG DES CHEMISCHEN SAUERSTOFFBEDARFS VON VERBRAUCHTEN METALLARBEITSFLÜSSIGKEITEN
CONSORTIUM DE MICRO-ORGANISMES ET SON UTILISATION POUR RÉDUIRE LA DEMANDE CHIMIQUE EN OXYGÈNE DE FLUIDE DE TRAVAIL DE MÉTAUX USÉ

(30) Priority: 30.07.2015 GB 201513431
(43) Date of publication of application: 06.06.2018
(73) Proprietor: Ford Motor Company Limited, Essex, CM13 3BW (GB)
(72) Inventor: POPE, William, Towcester Northamptonshire NN12 8RX (GB); AGER, Duane, Daventry Northamptonshire NN11 6UU (GB); GOODALL, Timothy, Brentwood Essex CM13 3BW (GB)
(74) Representative: Haseltine Lake Kempner LLP
(86) International application number: PCT/EP2016/068211
(87) International publication number: WO 2017/017263

(56) References cited:
- WO-A1-2008/102131
- WO-A2-01/04060
- WO-A2-2007/103875
- WO-A2-2011/104509
- WO-A2-2012/162518
- WO-A2-2014/033638

### SEQUENCES

### SEQUENCE LISTING

<110> Microbial Solutions Limited
<120> CONSORTIUM
<130> P128336PC00
<150> GB1513431.5
   <151> 2015-07-30
<160> 5
<170> PatentIn version 3.5
<210> 1
   <211> 1308
   <212> DNA
   <213> Rhizobium radiobacter NCIMB 42280 (Partial 16S ribosomal subunit)
<220>
   <221> misc_feature
   <222> (1)..(1308)
   <223> n = any of A, T, G, C
<400> 1
   nnnnnnnnnn nnnnnnnnnn nttcacnnnn nncgctgann ctaccgtggt tagctgcctc      60
   cttgcggtta gnnnnnnnnn nnnnggtaaa accaactccc atggtgtgac gggcggtgtg     120
   tacaaggccc gggaacgtat tcaccgcagc atgctgatct gcgattacta gcgattccaa     180
   cttcatgcac tcgagttgca gagtgcaatc cgaactgaga tggcttttgg agattagctc     240
   gacatcgctg tctcgctgcc cactgtcacc accattgtag cacgtgtgta gcccagcccg     300
   taagggccat gaggacttga cgtcatcccc accttcctct cggcttatca ccggcagtcc     360
   ccttagagtg cccaactcaa tgctggcaac taagggcgag ggttgcgctc gttgcgggac     420
   ttaacccaac atctcacgac acgagctgac gacagccatg cagcacctgt tctggggcca     480
   gcctaactga aggacatcgt ctccaatgcc cataccccga atgtcaagag ctggtaaggt     540
   tctgcgcgtt gcttcgaatt aaaccacatg ctccaccgct tgtgcgggcc cccgtcaatt     600
   cctttgagtt ttaatcttgc gaccgtactc cccaggcgga atgtttaatg cgttagctgc     660
   gccaccgaac agtatactgc ccgacggcta acattcatcg ttttacggcg tggactacca     720
   gggtatctaa tcctgtttgc tccccacgct ttcgcacctc agcgtcagta atggaccagt     780
   aagccgccnt ncgccactgg tgttcctccg aatatctacg aatttcacct ctacactcgg     840
   aattccactt acctcttnca tnntcnagat acccagtatc aaaggcagtt nccagagttg     900
   agctctggga nttcaccccc tgacttaaan ntccnnccta cgtgcgcttt acncccagta     960
   anttcnaaca ngnnnncccc ctncgnnntt accnnnggct gntgnncnaa ntnngcnggg    1020
   cttnnttcnt cngnnnnncg tcattatnnt tnnncggnna ngnnnctttn nnnnntnnng    1080
   nctttcatcn cnncnnnngc nnngnctgga nnnggcttgn gccnnntgtc nannannnnn    1140
   cnncnnnnnn nctnncnnnn nnnnanttng ngncngggtn nnnnnnnncn cannnnnnng    1200
   gctnnnnnnn nnnntnnnnn annnnnnnna tnnncnnnnn nntgnnnnnn gcnnttaccn    1260
   ccnanannnn ncntannnnn nannnnnggg gcnannnnnn nttnnnnn                 1308
<210> 2
   <211> 479
   <212> DNA
   <213> Bacillus subtilis NCIMB 42282 (Partial 16S ribosomal subunit)
<220>
   <221> misc_feature
   <222> (1)..(479)
   <223> n = any of A, T, G, C
<400> 2
   nnnnnnnnnn nngnnnnnnn nncnngnngt tagcggcgga cgggtgagta acacgtgggt      60
   aacctgcctg taagactggg ataactccgg gaaaccgggg ctaataccgg atggttgttt     120
   gaaccgcatg gttcaaacat aaaaggtggc ttcggctacc acttacagat ggacccgcgg     180
   cgcattagct agttggtgag gtaacggctc accaaggcaa cgatgcgtag ccgacctgag     240
   agggtgatcg gccacactgg gactgagaca cggcccagac tcctacggga ggcagcagta     300
   gggaatcttc cgcaatggac gaaagtctga cggagcaacg ccgcgtgagt gatgaaggtt     360
   ttcggatcgt aaagctctgt tgttagggaa gaacaagtac cgttcgaata gggcggtacc     420
   ttgacggtac ctaaccagaa agccacggct aactacgtgc cagcagccgc ggtaataca      479
<210> 3
   <211> 475
   <212> DNA
   <213> Bacillus thuringiensis NCIMB 42283 (Partial 16S ribosomal subunit)
<220>
   <221> misc_feature
   <222> (1)..(475)
   <223> n = any of A, T, G, C
<400> 3
   nnnnnnnnnn nnntgctctt anganttagc ggcggacggg tgagtaacac gtgggtaacc      60
   tgcccataag actgggataa ctccgggaaa ccggggctaa taccggataa tattttgaac     120
   tgcatggttc gaaattgaaa ggcggcttcg gctgtcactt atggatggac ccgcgtcgca     180
   ttagctagtt ggtgaggtaa cggctcacca aggcaacgat gcgtagccga cctgagaggg     240
   tgatcggcca cactgggact gagacacggc ccagactcct acgggaggca gcagtaggga     300
   atcttccgca atggacgaaa gtctgacgga gcaacgccgc gtgagtgatg aaggctttcg     360
   ggtcgtaaaa ctctgttgtt agggaagaac aagtgctagt tgaataagct ggcaccttga     420
   cggtacctaa ccagaaagcc acggctaact acgtgccagc agccgcggta ataca          475
<210> 4
   <211> 1252
   <212> DNA
   <213> Pseudomonas putida NCIMB 42441 (Partial 16S ribosomal subunit)
<220>
   <221> misc_feature
   <222> (1)..(1252)
   <223> n = any of A, T, G, C
<400> 4
   nnnnnnnnnn nnnnngnnnn nnnntgcagt cgagcggatg agaagagctt gctcttcgat      60
   tcagcggcgg acgggtgnnt aatacctagg aatctgcctg gtagtggggg acaacgtttc     120
   gaaaggaacg ctaataccgc atacgtccta cgggagaaag caggggacct tcgggccttg     180
   cgctatcaga tgagcctagg tcggattagc tagttggtga ggtaatggct caccaaggct     240
   acgatccgta actggtctga gaggatgatc agtcacactg gaactgagac acggtccaga     300
   ctcctacggg aggcagcagt ggggaatatt ggacaatggg cgaaagcctg atccagccat     360
   gccgcgtgtg tgaagaaggt cttcggattg taaagcactt taagttggga ggaagggcag     420
   taagcgaata ccttgctgtt ttgacgttac cgacagaata agcaccggct aactctgtgc     480
   cagcagccgc ggtaatacag agggtgcaag cgttaatcgg aattactggg cgtaaagcgc     540
   gcgtaggtgg ttcgttaagt tggatgtgaa atccccgggc tcaacctggg aactgcatcc     600
   aaaactggcg agctagagta gggcagangg tggtggaatt tcctgtgtag cggtgaaatg     660
   cgtagatata ggaaggaaca ccagtggcga angcgaccac ctgggctcat actgacactg     720
   aggtgcgaaa gcgtggggag caaacaggat tagataccct ggtagtccac gccgtaaacg     780
   atgtcaacta gccgttggaa tccttgagat tttagtggcg cagctaacgc attaagttga     840
   ccgcctgggg gagtacggnc gcaaggttaa aactcnaatg aattgacggg ggcccgcaca     900
   gcggnggagc atgtggttta atttcgaagc aacgcnaaga accttaccng gccntgacat     960
   ccnatgaact ttccnnanan ggattggtgc cttcggganc attganacag gngctgncat    1020
   nggntnntcg tcagcctcnn gtcntgagat ntngnttaag tcccntnacn nnnnnnancn    1080
   nnntncttta nttnccnncn nntnntggnn gnnnnntcta ngnnactgnc ngnnnnnaac    1140
   ccnnnnnnng nnnnnannnn cagtcntcnn ngnnnnnngn cnnngnnnnc nnnnngnnnn    1200
   naangnngnn nannnnccnn ncnnnnnnnn nnnnantnnn nnnaaaannn nn             1252
<210> 5
   <211> 461
   <212> DNA
   <213> Pseudomonas stutzeri NCIMB 42281 (Partial 16S ribosomal subunit)
<220>
   <221> misc_feature
   <222> (1)..(461)
   <223> n = any of A, T, G, C
<400> 5
   nnnnnnnnnn ctgctcntga ttcngcggcg gacgggtgag taatgcctag gaatctgcct      60
   ggtagtgggg gacaacgttt cgaaaggaac gctaataccg catacgtcct acgggagaaa     120
   gtgggggatc ttcggacctc acgctatcag atgagcctag gtcggattag ctagttggcg     180
   aggtaaaggc tcaccaaggc gacgatccgt aactggtctg agaggatgat cagtcacact     240
   ggaactgaga cacggtccag actcctacgg gaggcagcag tggggaatat tggacaatgg     300
   gcgaaagcct gatccagcca tgccgcgtgt gtgaagaagg tcttcggatt gtaaagcact     360
   ttaagttggg aggaagagca gtaagttaat accttgctgt tttgacgtta ccgacagaat     420
   aagcaccggc taacttcgtg ccagcagccn nnggtaatac a                         461

## Description

### FIELD OF THE INVENTION

The present invention relates to a novel consortium of micro-organisms that can be used in the treatment of industrial waste, methods and uses of the consortium and to bioreactors and treatment systems containing same.

### BACKGROUND

Metal working fluids (MWF) are an essential component of heavy manufacturing facilities (including automotive engine, transmission and stamping plants, aerospace industry and their supply chains). Specifically, they are used as coolants and lubricants for metal cutting and grinding, and drilling operations. MWF wastes contribute to the vast majority of organic compounds in wastewater produced by such manufacturing plants. In the UK, more than 400 million litres of waste MWF are produced annually, and worldwide figures are estimated to be more than 22.4 x 10⁹ litres annually. Once oil-based MWFs have become operationally exhausted, they need to be treated so they can be disposed of and to meet the requirements of European Union and US federal directives regulating effluent discharge.

One method for the treatment of MWFs comprises the biological treatment of MWFs in which micro-organisms are added to digest the unwanted constituents. Such methods of bioremediation of MWFs are often unable to reduce the chemical oxygen demand (COD) sufficiently without initial processing - such as filtration or ultra-filtration of the spent MWF (see, for example, van der Gast & Thompson (2005) Biotechnology & Bioengineering 89, 3 357-366), which adds considerable time, inconvenience and expense to the biological methods. Other biological methods that have been described to bring about some reduction in COD have utilised a liquid inoculation of microorganisms into a bioreactor, wherein the microorganisms are capable of reducing the COD content of spent MWF. For example, Muszynski & Lebkowska (2005) Polish Journal of Environmental Studies 14, 1 p73-79 and Hila et al. (2005) Journal of Chemical Technology and Biotechnology (2005) 80, 641-648 describe the selection and culturing of microorganisms from spent MWF and their subsequent liquid inoculation into a bioreactor. Another biological method that has been described to bring about some reduction in COD is to use a defined consortium of microorganisms. In this regard, WO2008/102131 describes the use of a consortium of microorganisms consisting of at least an *Rhizobium spp.,* a *Comamonas* spp., a *Methylobacterium* spp. and a *Microbacterium* spp. for treating spent MWF. Specifically, the consortium of microorganisms consisted of *Rhizobium radiobacter*, *Comamonas testosteroni*, *Methylobacterhim mesophilicum*, *Microbacterium esteraromaticum* and *Microbacterium saperdae.*

WO2011/104509 describes a method for treating spent metal working fluid (MWF), comprising the step of providing a biofilm of microorganisms on a solid support matrix. The microorganisms may be a consortium of two of more bacteria, for example such as disclosed in WO2008/102131.

There is a continuing need in the art for improved methods of reducing the COD content of industrial fluids or industrial effluents - such as spent MWFs. The present invention seeks to address this need.

### SUMMARY OF THE INVENTION

The present inventors have devised a novel consortium, combination or mixture of microorganisms which performs better at reducing the COD of MWFs than the consortium disclosed in WO2008/102131. The performance is better in that the COD can be reduced to lower levels with the present consortium as compared to the consortium described in WO2008/102131. The lower levels of COD reduction can also be achieved in a shorter period of time. Based on the data presented herein, it is estimated that the novel consortium can achieve around at least a further 25% reduction in COD levels as compared to the consortium described in WO2008/102131. The novel consortium may be used in the treatment of industrial fluids or industrial effluents or spent MWFs without any prior fractionation or separation or any other form of pre-treatment process being required.

Herein is described a consortium or a mixture or a combination of micro- organisms comprising, consisting or consisting essentially of Rhizobium spp., Bacillus spp., and Pseudomonas spp. The consortium can be for use in reducing the COD of spent MWF.

The Rhizobium spp. may comprise the sequence set forth in SEQ ID NO:1.

The Bacillus spp. may comprise the sequence set forth in SEQ ID NO:2 or SEQ ID NO:3.

The Pseudomonas spp. may comprise the sequence set forth in SEQ ID NO:4 or SEQ ID NO:5.

The Rhizobium spp. may be *Rhizobium radiobacter,* the Bacillus spp. may be *Bacillus subtilis* and/or *Bacillus thuringiensis,* and the *Pseudomonas spp.* may be *Pseudomonas putida* and/or *Pseudomonas stutzeri.*

The consortium of micro-organisms may comprise, consist or consist essentially of *Rhizobium radiobacter, Bacillus subtilis, Bacillus thuringiensis, Pseudomonas putida and*/*or Pseudomonas stutzeri.*

The consortium of micro-organisms may comprise, consist or consist essentially of *Rhizobium radiobacter* NCIMB 42280 and/or *Bacillus subtilis* NCIMB 42282 and/or *Bacillus thuringiensis* NCIMB 42283 and/or *Pseudomonas putida* NCIMB 42441 and/or *Pseudomonas stutzeri* NCIMB 42281.

The present invention provides a consortium of micro-organisms comprising, consisting of or consisting essentially of *Rhizobium radiobacter* NCIMB 42280, *Bacillus subtilis* NCIMB 42282, *Bacillus thuringiensis* NCIMB 42283, *Pseudomonas putida* NCIMB 42441 and *Pseudomonas stutzeri* NCIMB 42281, hereinafter referred to as 'the consortium of the invention'.

There is provided a biofilm comprising the consortium of micro-organisms. The present invention provides a biofilm comprising the consortium of the invention, wherein the biofilm is colonised on a solid support.

There is provided a method for reducing the chemical oxygen demand (COD) of a spent metal working fluid (MWF), comprising contacting the MWF with the consortium of micro-organisms or the biofilm. The present invention provides a method for reducing the COD of spent MWF, comprising contacting the MWF with the consortium of the invention, or the biofilm of the invention, or the bioreactor of the invention. Optionally, the method comprises the steps of: (a) providing the biofilm of the invention on a solid support matrix in a first bioreactor; (b) transferring at least a portion of the solid support matrix comprising the biofilm of microorganisms from the first bioreactor into a second bioreactor; and (c) incubating the microorganisms in the second bioreactor to reduce the COD of the spent MWF contained therein.

There is provided a bioreactor for reducing the COD of spent MWFs, the bioreactor comprising the consortium of micro-organisms or the biofilm. The present invention provides a bioreactor for reducing the chemical oxygen demand (COD) of spent metal working fluids (MWFs), the bioreactor comprising said biofilm comprising said consortium, wherein the solid support is fixed to the bioreactor. Optionally, at least a portion of the solid support is removable from the bioreactor. Optionally, the bioreactor further comprises spent MWF. The bioreactor may further comprise (i) a second solid support matrix, wherein said second solid support matrix is not or is not substantially colonised by a biofilm of microorganisms; and (ii) optionally, diluted spent MWF.

There is provided the use of the bioreactor in the reduction of the COD of spent MWF.

There is provided a method for reducing the COD of spent MWF, comprising contacting the MWF with the bioreactor.

There is provided a method for treating spent MWF, comprising the steps of: (a) providing the biofilm on a solid support matrix in a first bioreactor; (b) transferring at least a portion of the solid support matrix comprising the biofilm of microorganisms from the first bioreactor into a second bioreactor; and (c) incubating the microorganisms in the second bioreactor to reduce the COD of the spent MWF contained therein.

There is provided a method for preparing a biofilm of microorganisms that is capable of reducing the COD content of spent MWF comprising the steps of: (a) providing the biofilm on a solid support matrix in a first bioreactor; (b) transferring the solid support matrix comprising the biofilm of microorganisms from the first bioreactor into a second bioreactor; and (c) culturing the biofilm of microorganisms in the second bioreactor in the presence of spent MWF.

There is provided a bioreactor for treating MWF comprising: (i) a first solid support matrix comprising the biofilm, wherein said biofilm is capable of reducing the COD content of MWF, optionally wherein said biofilm has been established in a different bioreactor; (ii) a second solid support matrix, wherein said second solid support matrix is not or is not substantially colonised by a biofilm of microorganisms; and (iii) optionally, diluted spent MWF. By way of example, the solid support is not substantially colonised by the biofilm of microorganisms when less than about 10% of solid support matrix is not colonised by the biofilm of microorganisms, or less than about 9%, or less than about 8%, or less than about 7%, or less than about 6%, or less than about 5%, or less than about 4%, or less than about 3%, or less than about 2%, or less than about 1% or less than about 0.1%, or when a biofilm of microorganisms is not visible or is not detectable or is not present.

In the present invention, there is provided *Rhizobium radiobacter* NCIMB 42280.

In the present invention, there is provided *Bacillus subtilis* NCIMB 42282.

In the present invention, there is provided *Bacillus thuringiensis* NCIMB 42283.

In the present invention, there is provided *Pseudomonas putida* NCIMB 42441.

In the present invention, there is provided *Pseudomonas stutzeri* NCIMB 42281.

There is provided a method or a bioreactor or a use or a consortium or a biofilm as described herein with reference to the accompanying description and drawings.

In one embodiment, the second bioreactor is initially filled either before or after step (b) with spent MWF, suitably, diluted spent MWF, in which the COD thereof is between 5,000 to 10,000 mg/L.

In one embodiment, the solid support matrix comprises, consists or consists essentially of woven tubes of plastic.

In one embodiment, the air flow in the first and/or the second bioreactor is between 250 to 300 litres per minute per 5000 litres of liquid bioreactor volume.

In one embodiment, at least a portion of the spent MWF from the second bioreactor is used to inoculate one or more further bioreactors, optionally wherein the further bioreactor(s) comprise a solid support matrix which is not substantially colonised by microorganisms.

In one embodiment, said step is repeated one or more times to inoculate one or more further bioreactors.

In one embodiment, the biofilm of microorganisms on the first solid support matrix is capable of reducing the COD of spent MWF to 5,000 mg/L COD or less.

In one embodiment, 10% of the solid support matrix is the first solid support matrix and the remaining volume of the second bioreactor is occupied by the second solid support matrix and/or wherein said second bioreactor is reversibly connected to one or more further bioreactors to allow the passage of spent MWF therefrom, wherein the spent MWF has a COD of 5,000 mg/L or less.

The nucleotide sequences set forth in SEQ ID Nos 1-5 are also disclosed. A nucleotide sequence comprising, consisting or consisting essentially of the sequence set forth in SEQ ID Nos 1-5 is also disclosed.

A consortium, a biofilm, a method, a bioreactor or a use as described herein with reference to the accompanying description and drawings is also disclosed.

### BRIEF DESCRIPTION OF THE DRAWINGS

**Figure 1** shows a comparison of the performance of the consortium of the present invention (R4T) versus the consortium described in WO2008/102131 (Microcycle) on Hocut® 795B. Hocut® 795B is a high lubricity chlorine-free long-life soluble oil, widely available for purchase, from, for example from Houghton International). The upper figure shows the level of COD reduction across a time period of 11 days. The lower figure shows the same results presented as a percentage of initial COD removed.
**Figure 2** shows further comparisons of the performance of the consortium of the present invention (R4T) versus the consortium described in WO2008/102131 (Microcycle) on Hocut® 795B. Experiments were carried out over a period of 7 days or over a period of 10 days.
**Figure 3** shows a further comparison of the performance of the consortium of the present invention (R4T) versus the consortium described in WO2008/102131 (Microcycle) on a waste stream containing about 10% metal working fluid emulsion.
**Figures 4a to 4c** shows the NCIMB Deposit Receipt for *Rhizobium radiobacter* NCIMB 42280.
**Figures 5a to 5c** shows the NCIMB Deposit Receipt for *Pseudomonas stutzeri* NCIMB 42281.
**Figures 6a to 6c** shows the NCIMB Deposit Receipt for *Bacillus subtilis* NCIMB 42282.
**Figures 7a to 7c** shows the NCIMB Deposit Receipt for *Bacillus thuringiensis* NCIMB 42283.
**Figures 8a to 8c** shows the NCIMB Deposit Receipt for *Pseudomonas putida* NCIMB 42241.
**Figure 9** shows performance of an industrial scale bioreactor comprising the consortium of the present invention on effluent fluids from an automotive plant.

### DETAILED DESCRIPTION

COD is a measure of how much oxygen would be necessary to oxidise the components of materials such as waste effluents, and is generally considered to be a measure of the organic content of such materials. COD is measured in mg/L. The currently tolerated level for wastewater COD in the UK is 2000 mg/L, although it is probable that this level will be reduced.

As used herein, the term 'spent', as used in connection with MWFs, indicates a MWF after use. MWFs are generally provided as concentrates which must be diluted to between about 6% to 12% w/v in water prior to use. The methods described herein are suitable to treat the diluted concentrates without further treatment, such as ultrafiltration or additional dilution steps. Indeed, it is preferred that the MWF to be treated is not ultrafiltered.

As used herein, the term 'unprocessed' indicates that the MWF has not been filtered, ultrafiltered, fractionated, separated by any other means, chemically treated, or otherwise processed subsequent to normal use and prior to contact with the consortium. The spent MWF may be treated to enhance the ability of the consortium to reduce the COD of the spent MWF, or to facilitate handling thereof, and such treatment may involve wanning and/or buffering and/or dilution. Neutralisation by appropriate acid/alkali treatment to improve the environment for the consortium may be desirable. In general, such treatments are not necessary, and it is an advantage that spent MWFs can be used in the methods immediately after use and without any form of pre-treatment.

The consortium may be used in any suitable manner, and may be added directly, preferably as a culture, to the spent MWF, which may be treated with the consortium in vats, tanks or reservoirs, preferably with mechanical agitation while the COD is reduced. The spent MWF may also be processed together with the consortium in any other desired manner, such as by continuous processing through a series of tanks or reservoirs, or through pipes, and allowed to be released into the environment once the COD has reduced the desired levels. Consortium culture may be collected from the processed MWF for re-use, if desired.

It has been found that the consortium forms a particularly effective biofilm capable of reducing COD to low levels of 5000 mg/L or less.

It has been found that the consortium forms a particularly effective biofilm capable of reducing COD to low levels of 5000 mg/L or less in short periods of time.

A biofilm grown from the consortium described herein is capable of reducing the COD of Hocut® 795B by about 62% in about 6 days. A biofilm grown from the consortium described herein is capable of reducing the COD of Hocut® 795B by about 81% in about 9 days. A biofilm grown from the consortium described herein is capable of reducing the COD of Hocut® 795B by about 88% in about 10 days.

There is provided a method for reducing the COD of spent, preferably unprocessed, MWFs, comprising contacting the MWF with a biofilm, wherein the biofilm has at least three, four or five members which are selected from at least one each of Rhizobium spp., Bacillus spp., and Pseudomonas spp., suitably, said biofilm being capable of growth in untreated semi-synthetic MWFs.

As used herein, 'untreated' indicates that the MWF has not been diluted, filtered, ultrafiltered, or otherwise treated after its primary use in metal working and prior to being contacted with the biofilm.

The term 'biofilm' is used herein to describe a community of micro-organisms that, together, are capable of greater COD reduction of a given MWF, and preferably all MWFs, than could be achieved by the cumulative effect of each of the family members individually. For convenience, biofilms will generally be referred to hereinunder, but it will be appreciated that this includes reference to the consortia, unless otherwise apparent from the context.

Because biofilms are communities, rather than loose associations of individual micro-organisms, they are generally able to resist perturbations of conditions such as variation in temperature, pH or pollution load. Biofilms are self-sustaining under permissive conditions, and the biofilms are preferably selected to be self-sustaining in semi-synthetic MWF - such as Hysol X®. Permissive conditions will also preferably involve a temperature of between 10 °C and 37 °C and/or a pH of between 6 and 9, although temperatures and pHs outside of this range will frequently be sufficient to allow growth, but this may not be as great as when these parameters are in preferred ranges. Likewise, it is preferred that the biofilms are capable of growth on all commercially available MWFs, both when the MWFs have been prepared for use and once spent. It will be appreciated that the biofilms are particularly preferred for use with spent MWFs.

There is provided a bioreactor suitable to reduce the COD of spent, preferably unprocessed, MWFs, the bioreactor comprising a biofilm having at least three, four of five members which are selected from at least one each of Rhizobium spp., Bacillus spp., and Pseudomonas spp., said biofilm being capable of growth in untreated semi-synthetic MWFs.

There is provided a method for reducing the COD of unprocessed MWF waste, comprising contacting the MWF with a biofilm or bioreactor as described herein. This method is particularly suitable for synthetic MWFs, as well as semi-synthetic and oil-based MWFs.

MWFs to be treated by biofilms or bioreactors of the present disclosure are preferably unprocessed. Treatment steps may be employed as noted herein, and may involve warming if ambient temperatures might result in sluggish catabolism from the biofilm, such as where the temperature drops below about 10 °C, and/or dilution, preferably by water, to thin the sludge for better access to the bioreactor, for example.

It is an advantage that the bioreactors are self-sustaining and that, failing an occurrence such as a toxic event, the biofilm does not need replacing, even after a number of batches have been treated.

The term 'bioreactor' is used herein to describe apparatus adapted to support a biofilm and to enable the biofilm to be brought into contact with spent MWF. Such bioreactors may also be used for the treatment of any other liquid waste susceptible to degradation by the biofilms, but are primarily intended for the treatment of spent MWFs.

Bioreactors generally comprise one or more supports for the biofilm which may grow to form a film thereover, and wherein the support is adapted to provide a significant surface area for exposure to the MWF. The bioreactor will generally comprise a lumen or reservoir into which the MWF is introduced, with the biofilm being provided on the support throughout all, or a substantial part, of the lumen of reservoir. In either scenario, it is generally preferable to retain the MWF in the bioreactor for a period sufficient to bring the COD down to a target level. As described below, a suitable target level is 2,000 mg/L, or less, but any suitable target value may be selected. It may also be desired to operate bioreactors in sequence, such that the MWF is either cycled through bioreactors or fed through bioreactors in sequence until a target level COD is attained. As noted above, the bioreactors may also be adapted for continuous throughput. It will also be clear to the skilled individual that, as the biofilm grows, not only will it grow and spread to cover the available surface within the bioreactor, but that it will also permeate the MWF, so that the treated waste will contain a substantial amount of the consortium making up the biofilm. For this reason, it is important to avoid the use of pathogens in the biofilm as far as possible. Thus, the present disclosure further provides a biofilm and/or bioreactor as defined, wherein the biofilm contains substantially no detectable pathogens, and preferably no detectable pathogens at all.

It will be appreciated that the present disclosure provides the use of a bioreactor, as defined herein, in the reduction of the COD of spent MWF. The disclosure further provides apparatus for use as a bioreactor and a bacterial preparation suitable to seed said apparatus to provide a bioreactor. Further provided is waste liquid treated by a method or bioreactor, especially where said waste is spent MWF, and more especially where the COD of the waste is 2,000 mg/L or lower.

The present disclosure also provides a method for reducing the COD of spent MWFs, comprising contacting the MWF with a combination of micro-organisms capable of growth in untreated semisynthetic MWFs.

Spent MWFs are those that have been used, such as described above, and are appropriate for disposal. The methods described herein may be used on unused MWFs, but it will be appreciated that this will not normally be contemplated, as it would waste MWFs that could otherwise be used for their stated purpose.

While it is particularly preferred to use semi-synthetic and oil-based MWFs, whether they are spent or intended for disposal, the present disclosure is also applicable to synthetic MWFs. The COD of oil-based MWFs - such as Shell Dromus B® - can exceed 60,000 mg/L. Such high levels of organic contamination have also previously been associated with the inability to use any form of bioremediation, as the high pollution load prohibits growth.

The present disclosure provides a method for reducing the COD of an MWF having a COD of 15,000 mg/L or higher, and suitably 20,000 mg/L or higher, and most suitably even up to 95000 mg/L or 100,000 mg/L or higher, comprising contacting the MWF with a biofilm or bioreactor as described herein. This method is particularly suitable for synthetic MWFs, as well as semi-synthetic and oil-based MWFs.

It is a particular advantage that it is possible to reduce the COD of MWFs to below 5,000 mg/L, preferably, to below 2,000 mg/L, and preferably to below 1,000 mg/L, and most preferably to 500 mg/L or below.

The methods can also reduce toxicity of the MWF as measured by the *Vibrio fischeri* bioluminescence test.

The combinations or consortia of micro-organisms described herein may be used to reduce COD of any fluids having significant hydrocarbon content and high CODs. Such fluids include MWFs, preferably spent MWFs, from the automotive industry, the aerospace industry, and their supply chains. Such fluids can also include fluids from food products industries, beverage can manufacturing industries, airport apron de-icing glycol wastes, waste oil handling and processing, wind farm generator gearbox oils, oily naturally occurring radioactive materials (NORMs) such as from the oil exploration industry and fracking industry waste streams.

The combinations of micro-organisms used in the methods described herein are referred to herein as consortia, and each consortium comprises at least 3 bacteria as defined herein. There is no particular limit to the number of members that any consortium can have, but it is generally preferred to constitute a consortium for use in the methods described herein from individual preparations or cultures of the members of the consortium, in order that the members do not compete in the absence of the MWF. It will be appreciated, therefore, that restricting the numbers of members will provide a logistical advantage, although there should not be less than three members.

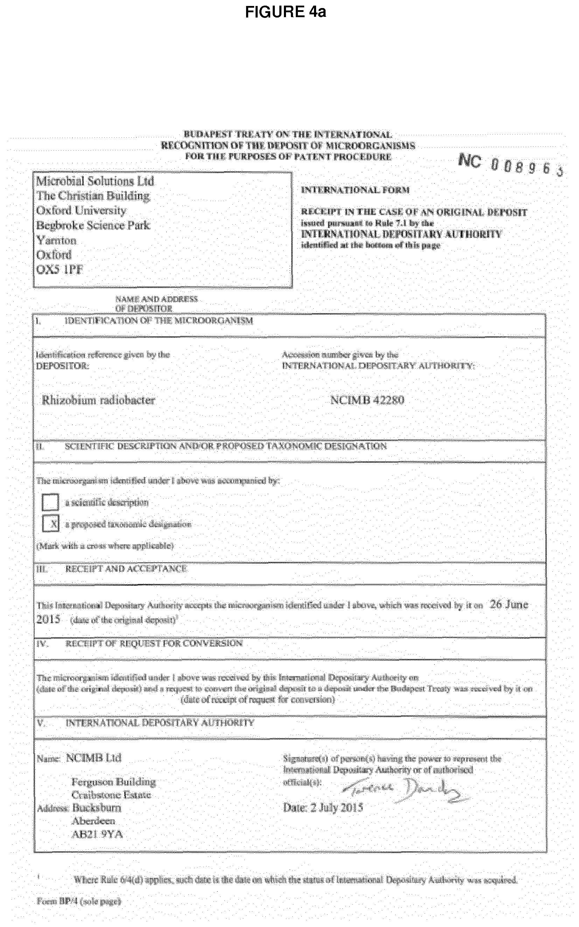

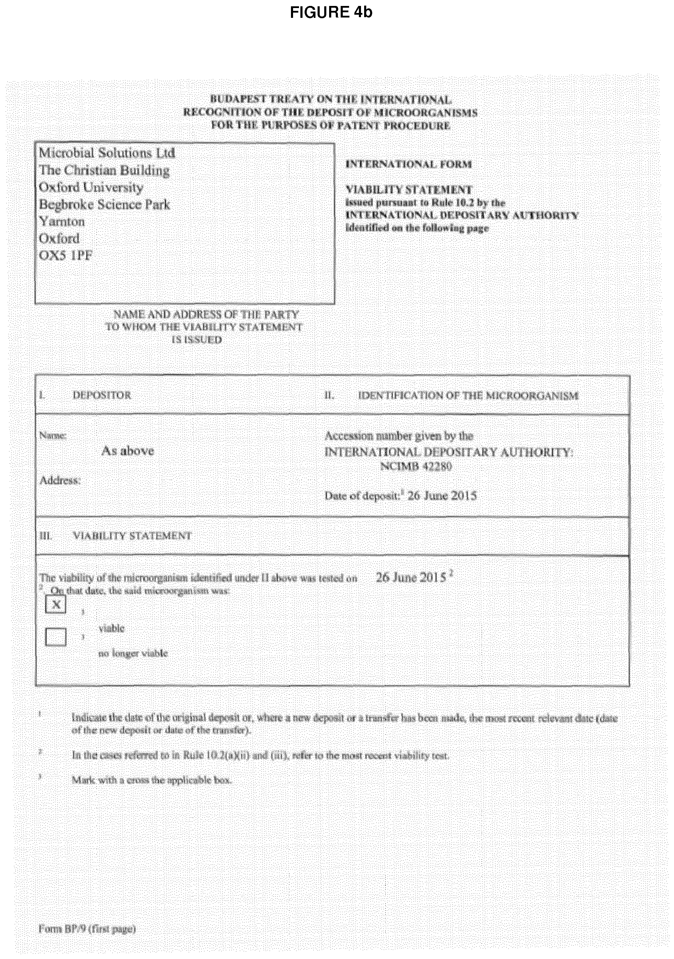

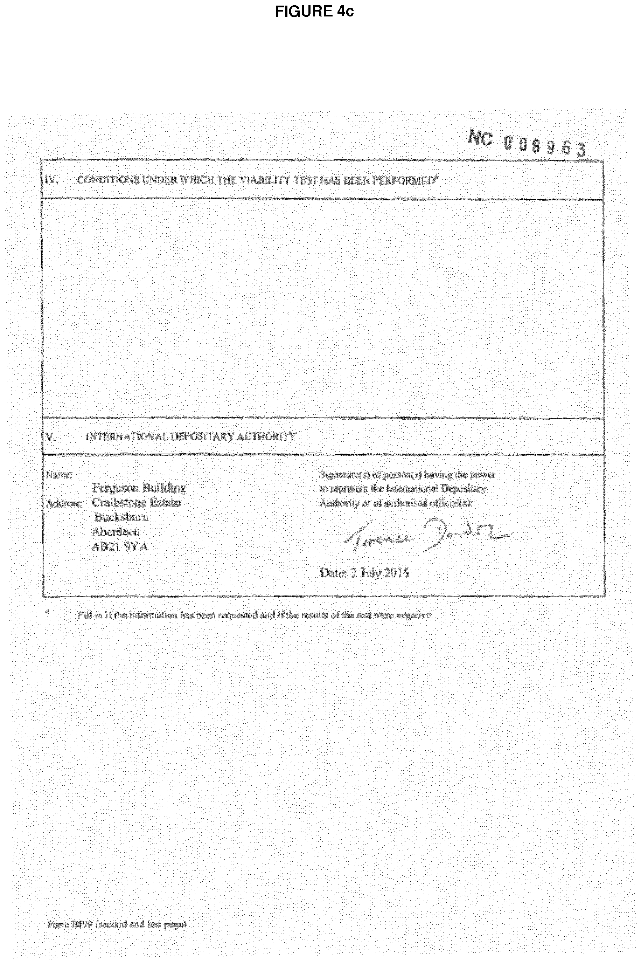

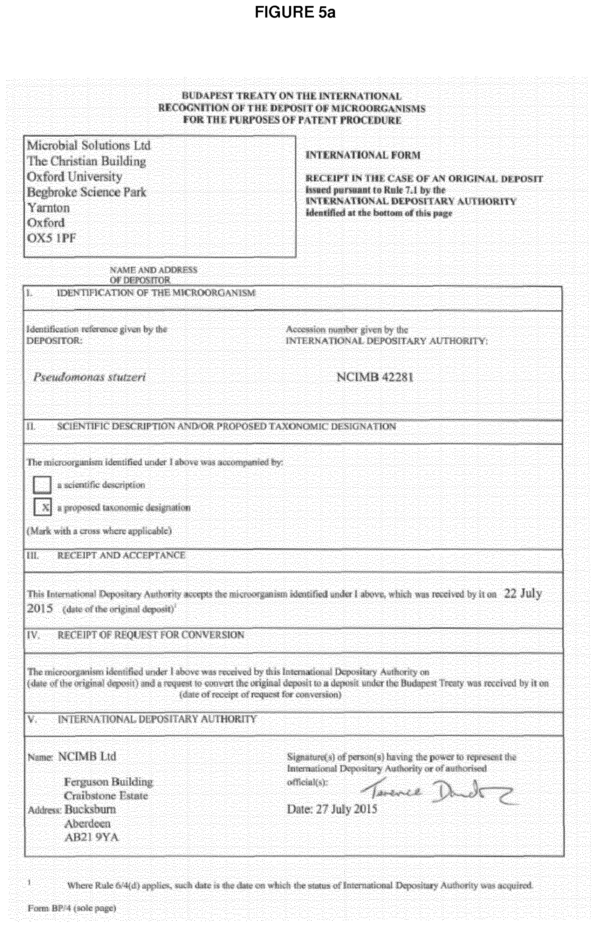

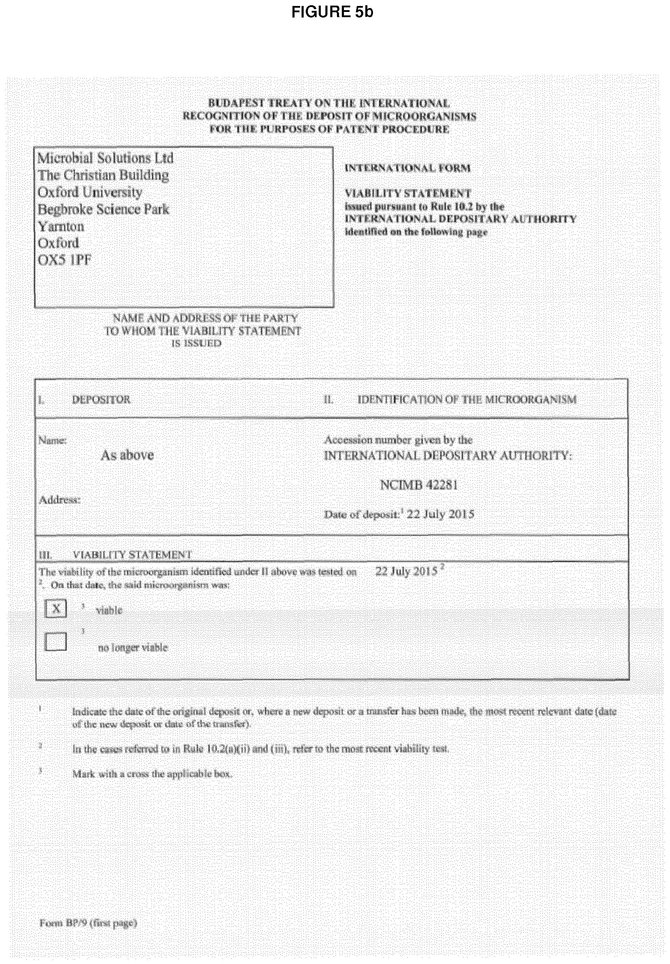

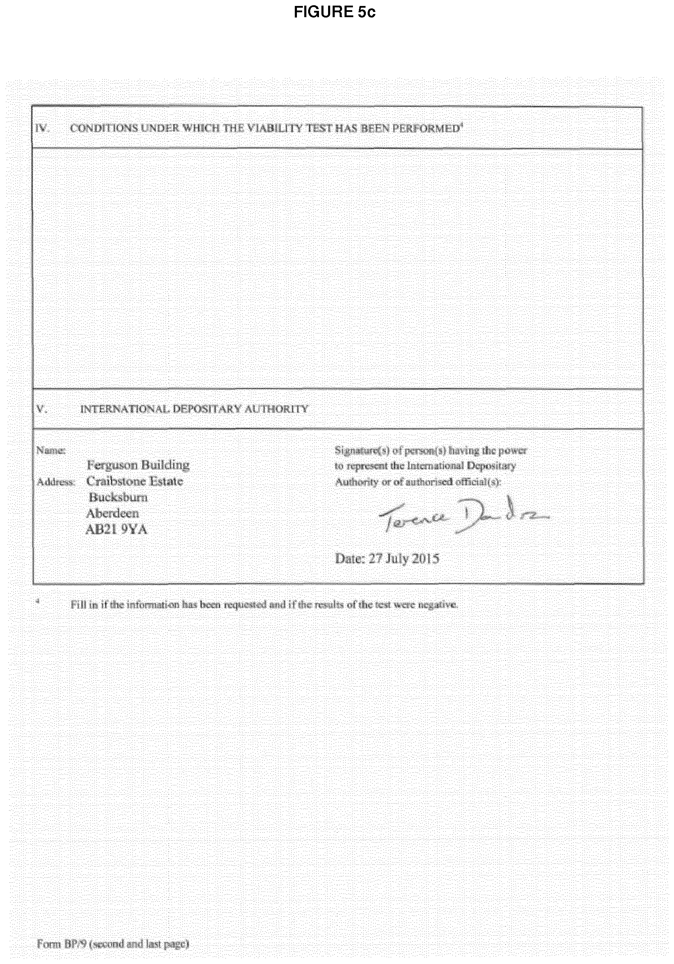

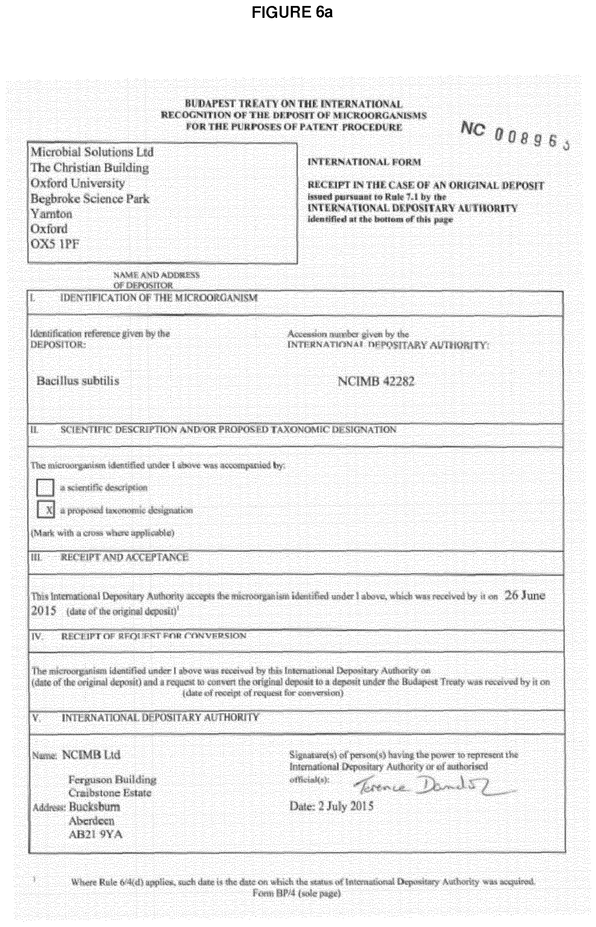

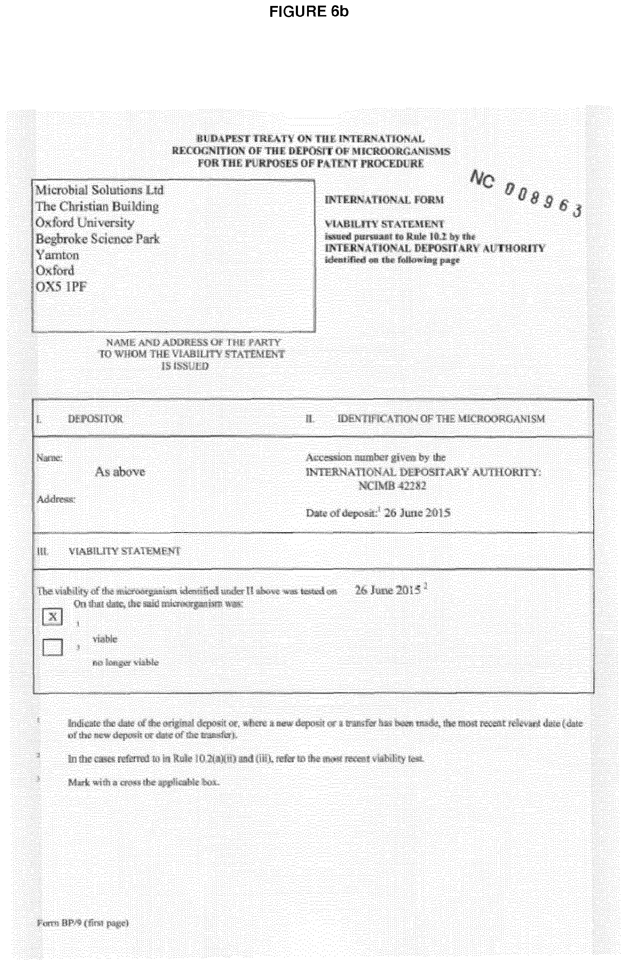

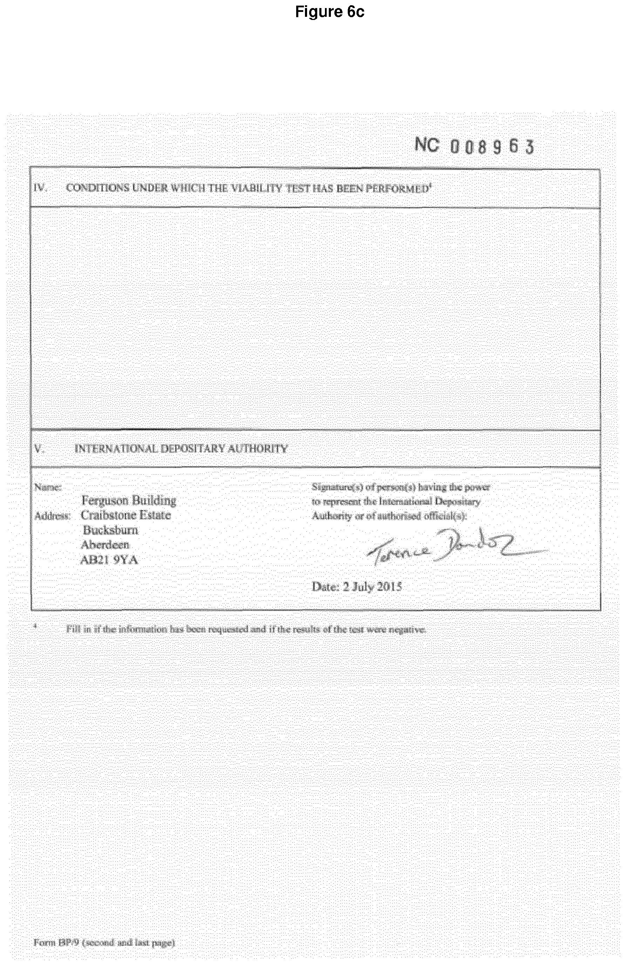

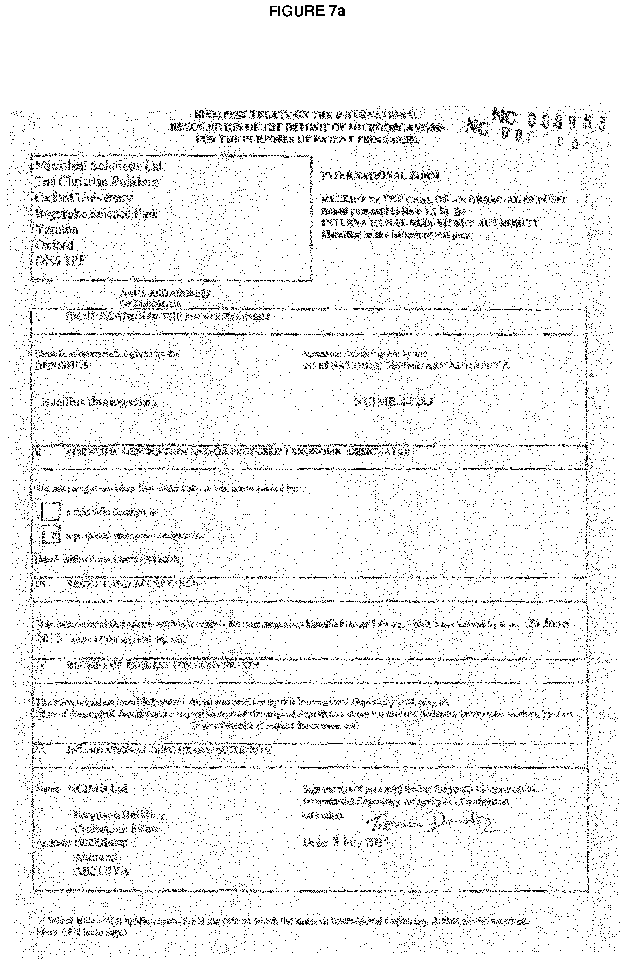

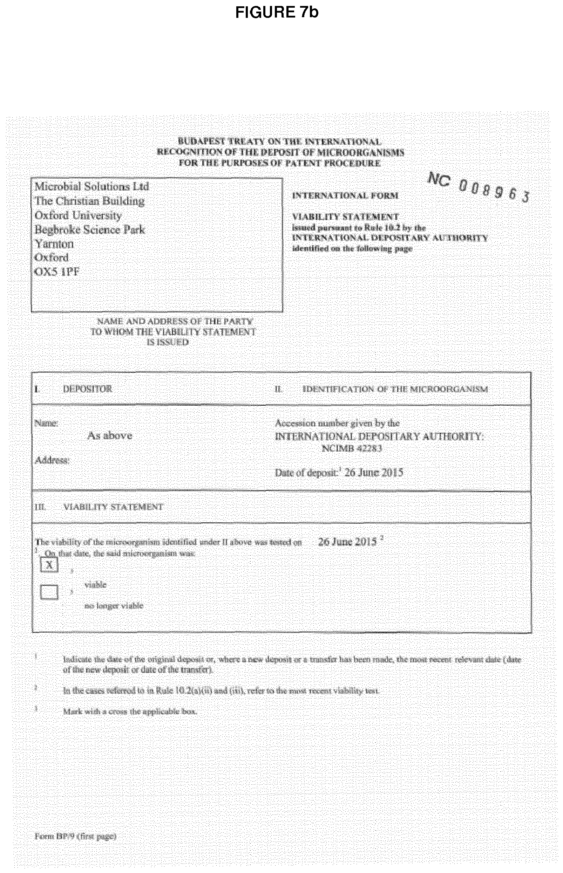

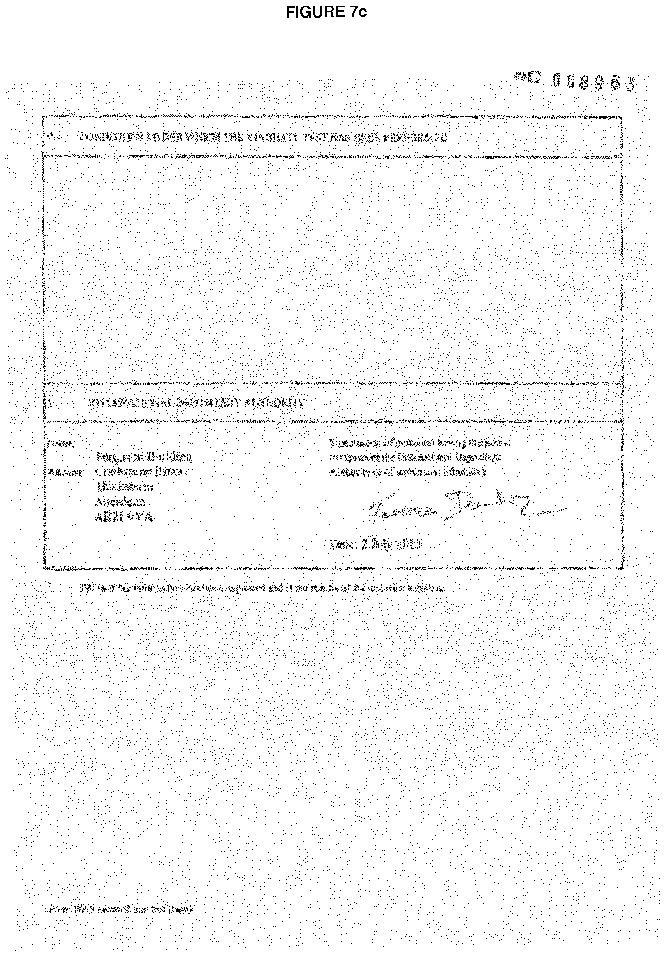

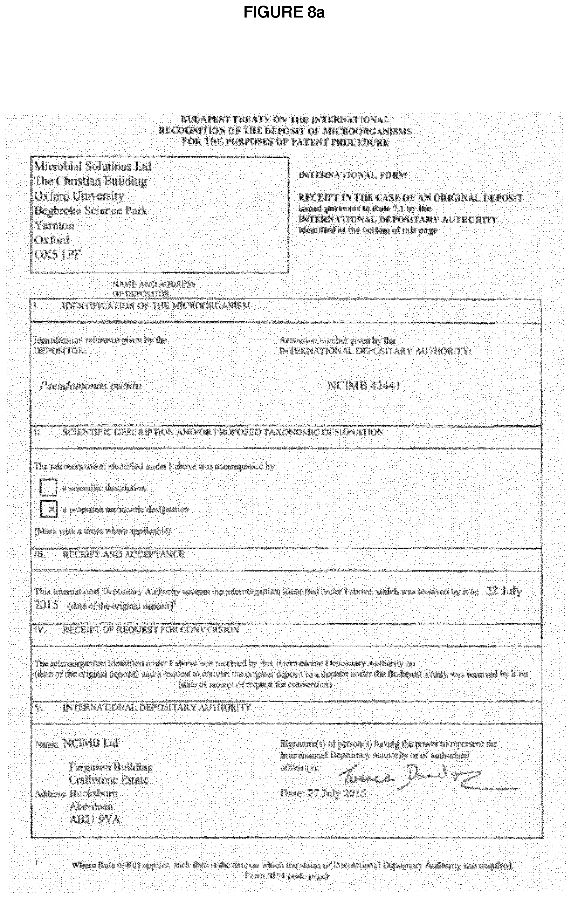

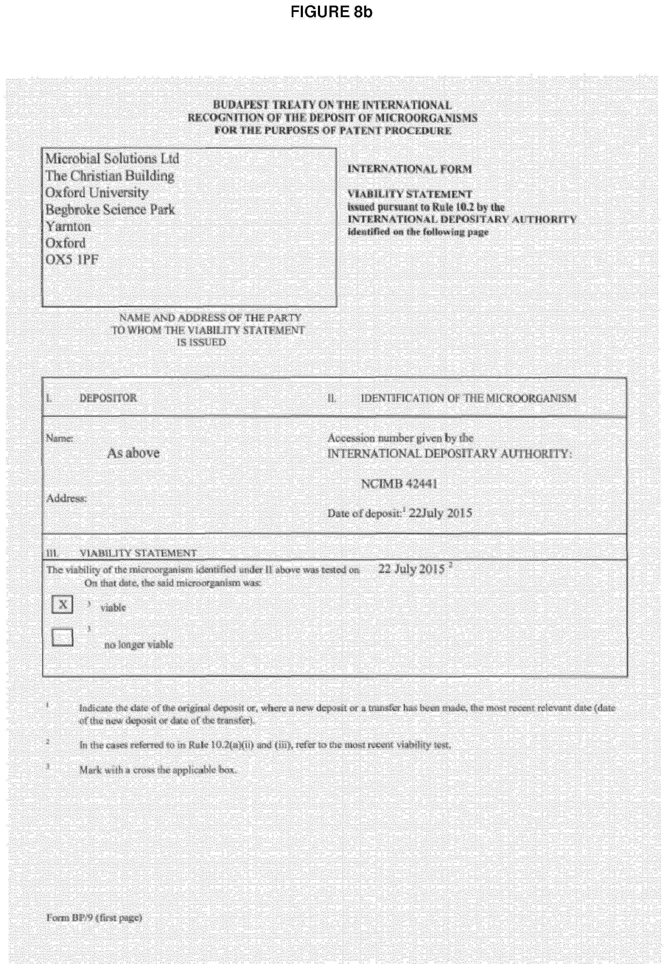

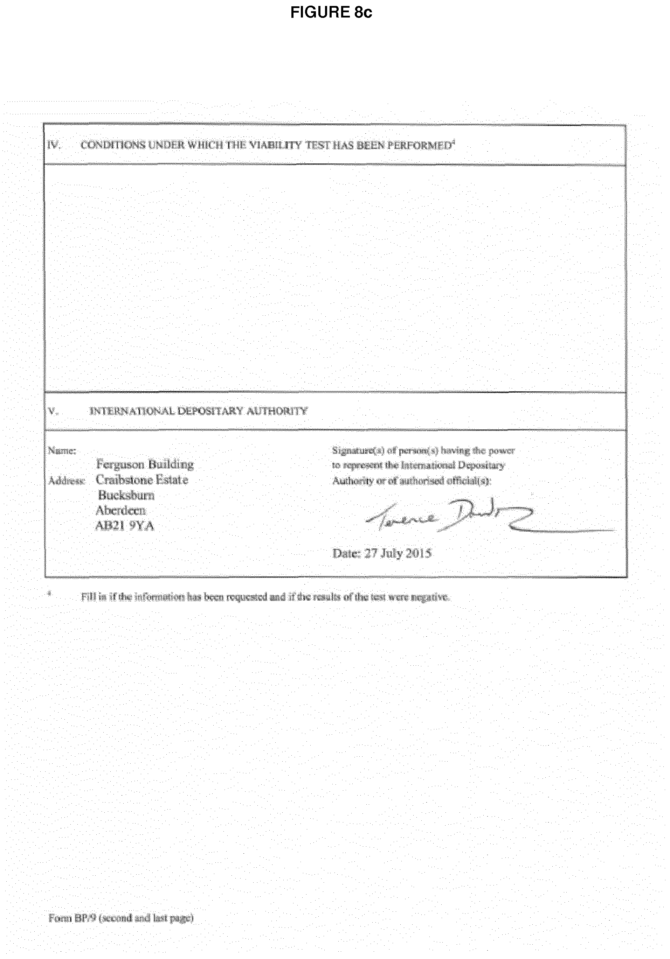

In the accompanying Examples, five bacteria constitute the consortium. These are *Rhizobium radiobacter, Bacillus subtilis, Bacillus thuringiensis, Pseudomonas putida* and *Pseudomonas stutzeri* isolated from MWF. Species of these bacteria have been deposited under the Budapest Treaty at NCIMB Ltd, Ferguson Building, Craibstone Estate, Bucksburn, Aberdeen, AB21 9YA, UK on 26 June 2015 or 22 July 2015 as follows:
- Rhizobium radiobacter - NCIMB 42280 - deposited on 26 June 2015;
- Bacillus subtilis - NCIMB 42282 - deposited on 26 June 2015;
- Bacillus thuringiensis - NCIMB 42283 - deposited on 26 June 2015;
- Pseudomonas putida - NCIMB 42441 - deposited on 22 July 2015; and
- Pseudomonas stutzeri - NCIMB 42281 - deposited on 22 July 2015;
The deposit receipts are shown in Figures 4 to 8.

It will be appreciated that the bacteria for inclusion in a consortium are selected from Rhizobium spp., Bacillus spp. and Pseudomonas spp., and mixtures thereof. It will be appreciated that any bacterium selected should be capable of growth in the MWF for treatment in the presence of the other members of the consortium, and preferably in the absence of other members of the consortium.

The consortium of micro-organisms can comprise at least one each of Rhizobium spp., Bacillus spp., and Pseudomonas spp. The consortium of micro-organisms can comprise at at least three, four or five micro-organisms selected from Rhizobium spp., Bacillus spp., and Pseudomonas spp. The Rhizobium spp. can be *Rhizobium radiobacter,* the Bacillus spp. can be *Bacillus subtilis* and/or *Bacillus thuringiensis,* and the *Pseudomonas spp.* can be *Pseudomonas putida* and/or *Pseudomonas stutzeri.* The consortium of micro-organisms can comprise, consist or consist essentially of the group consisting of *Rhizobium radiobacter, Bacillus subtilis, Bacillus thuringiensis, Pseudomonas putida* and *Pseudomonas stutzeri* or at least three, four or five members thereof. The consortium of micro-organisms can comprise, consist or consist essentially of *Rhizobium radiobacter, Bacillus subtilis, Bacillus thuringiensis, Pseudomonas putida* and *Pseudomonas stutzeri.* The consortium of micro-organisms can comprise, consist or consist essentially of *Rhizobium radiobacter* NCIMB 42280, *Bacillus subtilis* NCIMB 42282, *Bacillus thuringiensis* NCIMB 42283, *Pseudomonas putida* NCIMB 42441 *and Pseudomonas stutzeri* NCIMB 42281.

The methods will generally be carried out for a time and under conditions such that the COD is reduced to a desired level. The amount of time will depend on such parameters as the nature of the MWF, the starting COD level, the temperature and the pH, but will generally be between about 2 and about 10 days or more.

The MWF needs no treatment prior to contact with the consortium, although pre-treatments that are not excessively toxic, and preferably not at all toxic, to the biofilm may be applied if desired. After use, a waste, or spent, MWF will generally have a COD of around 100,000 mg/L or less, with a preferred average around 50,000-60,000 mg/L, and the consortia have proven capable of treating such MWFs. However, it may be desirable to dilute the MWF to assist in more rapid reduction of the COD, for example, or it may be desired to dilute the treated MWF.

The methods can be carried out over a range of pHs. MWFs often have a natural pH that is quite high, in the region of pH 9, and it has been established that the methods described herein are optimised around about a neutral pH, with a pH of between about 6 and about 7, inclusive, being desirable.

It is possible to warm the MWF during the treatment with the consortium, but this can prove expensive, and is not necessary. However, should it be desired to heat the spent MWF, then it is possible to use temperatures of up to 40 °C but preferably no higher than 37 °C, but temperatures of between 10 and 30 °C are generally acceptable with an optimal temperature of about 28 °C. Any temperatures outside of this range may be selected in accordance with the consortium used and the ambient conditions.

The method used to treat the MWF may be any that is suitable. For example, a preparation of the consortium may be added directly to the MWF and allowed to stand for a suitable length of time, such as two weeks. At the end of this time, a sample of the fluid can be taken in order to inoculate the next batch. The drawback with this process is that the efficacy of the consortium often tends to be reduced after a number of cycles, and further culture must be added. The process can be assisted by stirring and/or aeration. This process may be used to seed a bioreactor for example.

It is preferred to use bioreactors and, as described above, these may take any standard form. Preferred bacteria have been found to grow on and coat standard supports without any special conditions. However, it is generally advantageous to prepare the desired consortium and then to expose the consortium to the support. It is particularly preferred to mix the consortium with growth medium, which may be the MWF when first seeding the bioreactor.

The methods may be practised on MWFs, particularly oil-based MWFs, that have not been fractionated or filtered or, preferably, in any other way pre-treated prior to treatment, thereby substantially reducing expense and inconvenience.

It will be appreciated that the methods can be used for the treatment of any MWF, or any other industrial effluence of a similar nature, but that it is particularly advantageous that untreated oil-based MWFs can be treated. The bioreactor can be used to treat oil-based MWFs, particularly an untreated and most preferably an unfiltered, oil-based MWF.

The method of solid matrix inoculation may be used to inoculate a bioreactor as described in WO2011/104509. In solid matrix inoculation, once the biofilm has matured in a first bioreactor and is capable of reducing the COD of the spent MWF to the desired level, at least a portion of the solid matrix containing biofilm is removed from the first bioreactor and then transferred to a second bioreactor that is to be inoculated. The biofilm may be transferred from the first bioreactor to the second bioreactor immediately or the biofilm may be incubated for a period of time between the transfer. The incubation time and conditions will be chosen such that the viability of the biofilm is not substantially altered.

According to one embodiment, the volume of the solid-matrix that is transferred between the bioreactors is approximately at least about 0.5%, 1%, 2%, 3%, 4%, 5%, 6%, 7%, 8%, 9%, 10%, 15% or 20 % or more of the volume of the second bioreactor. In one embodiment, the volume of solid-matrix that is transferred is approximately at least about 5% or at least about 10% of the volume of the bioreactor to be inoculated. Thus, for example, 500 ml of solid-support matrix is introduced into a 5 litre lab bioreactor or 1000 litres of solid-support matrix is introduced into a 10,000 litre site reactor. Suitably, the remaining volume of the second bioreactor is occupied by solid-support matrix which does not have a biofilm of microorganisms thereon. Accordingly, the remaining volume of the bioreactor is occupied by solid support matrix that is free or substantially free of microorganisms. Thus, for example, the solid support matrix may be a new solid support matrix or it may be a cleaned solid support matrix upon which the amount of micro-organism growth is substantially absent.

Suitably the solid support matrix of the second bioreactor may therefore comprise at least about 50%, 60%, 70%, 80%, 90%, 95% or 100% of the remaining volume of the second bioreactor. The second bioreactor may not include any spent MWF at the time of transfer. Alternatively, the second bioreactor may already comprise the spent MWF prior to transfer or at least a portion of the spent MWF prior to transfer. Operating conditions in the second bioreactor are such that the solid support matrix in the second bioreactor becomes colonised by microorganisms to become covered with a biofilm over a period of days or weeks, preferably days, depending on, for example, the toxicity of the waste stream and the method of inoculation. Thus, for example, if the second bioreactor is liquid inoculated then typical maturation times are typically about 70 days or more; if the second bioreactor is solid matrix inoculated then maturation times are typically about 30 days or less. During the early commissioning period of the first and/or second bioreactors, the bioreactors should desirably be fed dilute spent MWF of between about 5000 to about 10,000 mg/L COD. Spent MWF with a higher COD may also be used - such as spent MWF of between about 5,000 to about 20,000 mg/L COD, of between about 5,000 to about 30,000 mg/L COD, of between about 5,000 to about 40,000 mg/L COD or between about 5,000 to about 50,000 mg/L COD. Suitably, the spent MWF that is added to the bioreactor(s) is diluted in order to reach this desired COD level. This is to minimise the toxic shock imposed by toxic components that may be present in the waste stream - such as biocides. According to one embodiment, the first and/or second bioreactors may be supplemented with a growth supplement - such as tryptic soya broth or a trace element solution (for example, a seaweed based trace element solution) and the like to help to establish the growth of the microorganisms into a biofilm in the bioreactor(s). Typically, this supplementation occurs at low levels - such as at about 1 to 10 µl per litre of bioreactor volume. The COD level of the spent MWF can be increased as the microorganisms present in the biofilm become accustomed to the increasing toxicity of the waste stream. As the biofilm on the solid matrix support in the bioreactor grows, it will spread and grow to cover any remaining solid support that has not been populated by the biofilm. The biofilm may also cover other available surfaces within the bioreactor. Biofilm may also slough dead and active cells into the liquid MWF. When removed the suspended biomass typically represents between about 500 mg/L to about 1500 mgl/L of COD.

The first and/or second bioreactors can be commissioned at various temperatures - such as room temperature (which is typically about 18 to 20°C) although lower temperatures are possible - such as temperatures as low as 12 °C. Once the bioreactor(s) have matured and are able to reduce the COD content spent MWF to the desired level then temperatures of between about 1 °C to about 35°C can be used. The operating temperature may exceptionally fall outside of this range, depending on the microorganism composition of the biofilm. Suitably, a temperature of between about 25°C to about 35 °C is used; more suitably, a temperature of between about 26°C to about 34 °C is used; more suitably, a temperature of between about 26 °C to about 33 °C is used; more suitably, a temperature of between about 26 °C to about 31 °C is used; more suitably, a temperature of between about 26 °C to about 30 °C is used; more suitably, a temperature of between about 27 °C to about 29 °C is used; most suitably, a temperature of about 28 °C is used. This temperature typically allows the COD reduction to be sustained at a high level for several days.

After treatment in the first and/or second bioreactor, the spent MWF will typically have a COD content of less than about 3,000 mg/L, more suitably, less than about 2,500 mg/L, more suitably, less than about 2,000 mg/L, or more suitably, less than about 1,500 mg/L.

The effluent from the second bioreactor can be used to inoculate one or more further bioreactors. Typically, the third bioreactor will comprise a solid support matrix which is not substantially colonised by microorganisms so that the effluent that is introduced therein can colonise the solid support matrix. This step can repeated one or more times to inoculate one or more further bioreactors. According to one embodiment, the second bioreactor may be reversibly connected to one or more further bioreactors to allow the passage of spent MWF therefrom. Suitably, the passage of the spent MWF is controllable such that it can be transferred once the COD of the spent MWF in the second bioreactor has reached 2000 mg/L or less. According to another embodiment, the further bioreactors may be manually filled with effluent from the second bioreactor. Suitably, the further bioreactors will also comprise a solid support matrix that is substantially free of microorganisms such that they can be colonised by the spent MWF introduced therein. Suitably, at least about 50%, 60%, 70%, 80%, 90% or 100% of the volume of the further bioreactor(s) will be occupied by the solid support matrix that is substantially free of microorganisms.

The solid support upon which the biofilm is grown in the bioreactor may be fixed to the bioreactor and/or removable from the bioreactor. Suitably, at least a portion of the solid support is removable from the bioreactor to allow the transfer of the solid support between bioreactors. The solid support can be any solid support that is suitable for establishing a biofilm. The solid support may be formed of plastic - such as polypropylene, metal, natural fibers - such as cotton and combinations thereof. The solid support may be formed of and/or include a coating formed of a hydrophobic material, such as polyethylene. Suitably, the material selected to form the solid support does not substantially degrade in the presence of MWF. The solid support may be substantially planar, substantially cylindrical, substantially conical, substantially spherical, substantially rectangular, substantially square, substantially oval shaped, and/or irregularly shaped. Suitably, one or more microorganisms are able to couple to the solid support in a bioreactor to form the biofilm, which is capable of being transferred from one bioreactor to another. Suitably, the microorganisms forming the biofilm cannot substantially slough off the solid support during use. Examples of solid supports include, but are not limited to, a biotower, a rotating biological contactor, rough stones, slats, plastic media, a reticulated foam particle, a microcarrier and/or media particles, diatomaceous earth, silica, alumina, ceramic beads, charcoal, or polymeric or glass beads and the like.

A preferred type of solid support comprises or consists of a plastic net - such as an extruded polyethylene net. Another preferred type of solid support comprises woven tubes of plastic - such as polypropylene - which provide a high surface area for biofilm growth whilst still allowing adequate liquid flow over the biofilm surface. Another preferred type of solid support a roughened surface to increase bacterial adhesion. Another preferred type of solid support comprises a combination of one or more, for example all, of these features.

In another embodiment, the solid support matrix comprises, consists or consists essentially of tubes of plastic- such as woven tubes of plastic (for example, polypropylene). The tubes of plastic may comprise about 200 net tubes, suitably, with an approximate 70 mm diameter in a length of 1 m. Each net tube typically comprises about 30 polyethylene strings with a diameter of about 2-3 mm. The net strings can be welded together so that they form square holes in the tube wall. The size of the holes is about 8mm x 8mm. These strings give a total area of about 100 m²/m³ in dry condition. Another format of net tubes comprises about 300 net tubes, suitably, with a diameter of about 50mm in a length of 1m. Each net tube typically comprises about 30 polyethylene strings with a diameter of about 2-3mm. The net strings can be welded together so that they form square holes in the tube wall. The size of the holes is about 4mm x 4mm. These strings give a total area of about 150 m²/m³ in dry condition. Another format of net tube has an outer diameter of about 50mm. Each cubic metre consists of about 300 net tubes with about 50mm diameter in a length of 1m. Each net tube comprises about 30 polyethylene strings with a theoretic diameter of about 3-4mm. The net strings are welded together so that they form square holes in the tube wall. The size of the holes is approx. 3mm x 3mm. These strings give a total area of about 200 m²/m³ in dry condition. Solid matrix is commercially available.

The bioreactors that are described herein may include a controller. A controller may be configured to automate the system. The controller may measure various parameters of the system, such as pressure; temperature; pH; COD content, a wastewater stream, and/or an outlet stream; an amount, type, and/or ratio of types of bacteria in the bioreactor; flow rates, air bubble stream; and/or a volume of water and the like. The controller may use measurements of the various parameters to modify values of one or more parameters of the system. The controller may measure and/or modify parameters of the system continuously or periodically. The bioreactor may be adapted for batch or continuous operation. The bioreactor may be an aerobic bubble column bioreactor. The bioreactor may be formed of plastic, metal, and/or other materials. The bioreactor may include one or more coatings. The coating may inhibit corrosion and/or facilitate removal of solids from a container. For example, a bioreactor may have a polytetrafluoride coating to inhibit corrosion and to inhibit solids from adhering to the bioreactor. The footprint of the bioreactor may be substantially square, substantially circular, substantially oval, substantially rectangular, and/or irregularly shaped. The bioreactor may have a shape configured to minimise stagnant regions in the bioreactor. In certain embodiments, the shape of the inner surface of the bioreactor may minimize stagnant regions in the container during mixing. The inner surfaces of the bioreactor may be rounded instead of meeting at an edge. For example, the inner surface of the bioreactor may have a shape substantially similar to an oval or a circle to minimize the presence of stagnant regions in the bioreactor, during use. In one embodiment, the bioreactor may have a shape in which substantially all of the liquid in one or more of the bioreactors circulates when mixed with a stirrer during use. The bioreactor may include one or more stirrers to agitate the spent MWF and/or gases in the bioreactor. One or more stirrers may be positioned to reduce dead mixing zones in the bioreactor. For example, the bioreactor with an oval cross-sectional area may include two stirrers approximately equally spaced across a bottom surface to inhibit areas of stagnation in the bioreactor. The bioreactor may include one or more inlets for wastewater streams, air bubble streams, and/or bacteria. The bioreactor may include one or more outlets for removal of liquids and/or solids from the bioreactor. Filters may be coupled to inlets and/or outlets. A filter and or a gravity trap may be coupled to an inlet to remove and/or break apart large solids. A filter may be coupled to an outlet to prevent solids such as waste solids, microorganisms, biofilm, and/or particulate matter from flowing out of the bioreactor. In one embodiment, a filter may inhibit contaminants from water flowing out of the bioreactor. For example, filter paper or an activated carbon filter may be coupled to an outlet to remove contaminants from a stream flowing out of the bioreactor. In certain embodiments, an electrocoagulation system may be coupled to inlets and/or outlets. An electrocoagulation system may be used prior to allowing spent MWF to enter the bioreactor comprising a biofilm and/or after allowing spent MWF to leave the bioreactor that includes a biofilm. The electrocoagulation system may cause compounds to precipitate and float to a top or bottom surface of the bioreactor for removal. In one embodiment, an electrocoagulation system may charge ions in the spent MWF. The charged ions may bind to oppositely charged ions and form a precipitate. Then the precipitates may float to a top surface or sink to a bottom surface of the bioreactor for removal from the spent MWF. In an embodiment the precipitates may be filtered out of the spent MWF.

Suitably, the first solid support matrix has been previously prepared in the first bioreactor as described herein. Suitably, the biofilm of microorganisms on the first solid support matrix is capable of reducing the COD of spent MWF to about 5000 mg/L COD or less, to about 4000 mg/L COD or less, to about 3000 mg/L COD or less, to about 2000 mg/L COD or less, to about 1000 mg/L COD or less, or to about 500 mg/L COD or less. Suitably, the volume of the first solid support matrix comprising the biofilm of microorganisms is at least about 1%, 2%, 3%, 4%, 5%, 6%, 7%, 8%, 9% or about 10% of the volume of the bioreactor. Suitably, the volume of the first solid support matrix comprising the biofilm of microorganisms is at least about 5% of the volume of the bioreactor. More suitably, the volume of the first solid support matrix comprising the biofilm of microorganisms is at least about 10% of the volume of the bioreactor. Suitably, the remaining volume of the bioreactor is occupied by a solid support matrix upon which a biofilm of microorganisms is not or is not substantially present. Suitably, the first and second support matrixes are moveable in and out of the bioreactor. More suitably, the first support matrix is moveable in and out of the bioreactor and the second support matrix is fixed within the bioreactor.

Once the desired COD level has been reached, the grey water remaining from the treatment can be released into the sewer or it can be used to inoculate further bioreactors. Grey water may optionally be further treated before release in order to kill any residual microorganisms. Suitable treatments include, but are not limited to ozone, irradiation heat, or any other treatment that does not increase the toxicity of the grey water. At this stage, a new batch of spent MWF can be introduced into the reactor for processing.
The following Examples are for illustrative purposes, and are not limiting on the invention in any way.

### EXAMPLES

### Example 1 - Materials and Methods

### Growth of bacterial consortia

Five bacteria are selected to constitute a consortium. These are *Rhizobium radiobacter* (NCIMB 42280), *Bacillus subtilis* (NCIMB 42282), *Bacillus thuringiensis* (NCIMB 42283), *Pseudomonas putida* (NCIMB 42441) *and Pseudomonas stutzeri* (NCIMB 42281). These five bacteria have been deposited at the NCIMB under the Budapest Treaty as described herein. The NCIMB Deposit Receipts are presented in Figures 4 to 8.
The novel combination of *Rhizobium radiobacter* (NCIMB 42280), *Bacillus subtilis* (NCIMB 42282), *Bacillus thuringiensis* (NCIMB 42283), *Pseudomonas putida* (NCIMB 42441) *and Pseudomonas stutzeri* (NCIMB 42281) is referred to herein at 'R4T'.
The combination of *Rhizobium radiobacter* (NCIMB 41462), *Comamonas testosteroni* (NCIMB 41463), *Methylobacterium mesophilicum* (NCIMB 41464), *Microbacterium esteraromaticum* (NCIMB 41465) and *Microbacteriumm saperdae* (NCMB 41466) as described in WO2008/102131 is referred to herein as 'Microcycle'.

Each strain is grown on culture plates, and a single colony extracted and incubated over night at 28 °C, before being plated out and cultured again (overnight in a static incubator at 28 °C) to ensure strain purity. The procedure is repeated as many times as necessary to confirm no other morphologies, but on average one "streak-back" procedure is sufficient. Bacteria are harvested using aseptic techniques. About 50 mg of bacterial biomass is resuspended in a storage cryotube with about 8 ml of glycerol and about 1 ml trypticase soy broth (tsb). Samples are then stored in a freezer at below -70 °C. To raise a culture from storage, the cryotube is removed from the freezer, kept on ice, and a sterile loop used to extract the bacteria for plating out on culture plates which are incubated overnight at 28 °C.

### Identification of bacterial consortia

After enrichment steps in minimal broth with MWF as the sole carbon source, bacterial isolates are identified by partial nucleotide sequencing of the 16S ribosomal subunit DNA. Examples of sequences from each species of the R4T consortium are described below under the heading 'Sequences'.

### Rapid MWF component utilisation screening

The ability of bacteria isolated from MWF to assimilate components of the fluid as sole carbon sources is assessed by inoculating each isolate into microtitre plate wells containing 100 µl M9 minimal media and 3 % v/v semi synthetic or oil based MWF as sole source of carbon. The master microtitre plates are incubated at 28°C overnight or until media broth became turbid. Bacteria are transferred from the master microtitre plates to flat bottom 96 well microtitre plates containing M9 minimal media and with individual synthetic MWF components (formaldehyde based biocide, benzotriazole, citric acid, formaldehyde, monoethanolamine, morpholine and triethanol amine) added at 5 mM concentration, using 200 µl tips arranged to the same pattern as the wells in the microtitre plates. The plates are covered with Seal-plate film (Sigma, Poole, UK) to prevent cross contamination or evaporation and incubated for 7 days at 28 °C. Optical density is measured every 24 hours at a wavelength of 620 nm using a *LUCY 1* spectrophotometer. This method allows a rapid screen of all 300 bacterial isolates for tolerance and ability to assimilate individual MWF components as the carbon source.

The isolated strains are inoculated separately into 250 ml conical flasks containing 100 ml of tryptic soy broth (10 % v/v. Difco, UK) and pre-filtered (using a 0.2 µm pore size filter, Millipore, UK), MWF wastewater (3 % v/v). The individual cultures are incubated at 28 °C in an orbital shaker for 12 hours (cell counts approximated to 10⁷ cells ml⁻¹). The cell suspensions are removed and resuspended in MWF wastewater, mixed together and added as a 10 % v/v inoculum into the bioreactors.

### Inoculation Conditions

The bacterial consortium is reconstituted by inoculating the individually stored strains separately into 250 mL conical flasks containing 50 mL of tryptic soy broth (10% v/v, Difco, U.K.) and fresh MWF concentrate (3% v/v). The monocultures are then incubated at 28 °C in an orbital shaker for 12 h. The cell suspensions are removed by centrifugation and resuspended in MWF effluent, mixed together, and added as a 10% v/v inoculum into the bioreactors. The bioreactors, after centrifugation, are inoculated with 3 g of wet esuspended biomass as described above.

### MWF

Semi-synthetic MWF can be a nutrient source. The fluid comprises various chemical constituents including an acid amine base, a lubricity package (oil and emulsifiers) and a biocide package. It is supplied as a concentrate and is typically diluted with water to form a 6 % v/v working fluid prior to use in machining operations.

### Solid matrix inoculation

A mature biofilm which has been grown on a solid support matrix is transferred from a first bioreactor into a second bioreactor. The bioreactor from which the biofilm is sourced has been consistently reducing the COD of spent MWF to about 2000 mg l-1 COD or less for greater than about 1 week for continuous flow or for a minimum of 2 batch runs in batch mode. The mature biofilm can be sourced from bioreactors that were originally liquid inoculated and have undergone the lengthy maturation process in a laboratory for example, or they can be sourced from a bioreactor previously propagated by solid-matrix-transfer of a mature biofilm. If culture isolates are used for establishing the biofilm then the inoculum can comprise a bacterial community selected from operational MWFs on 1/10 tryptic soya broth with the addition 1-5% MWF or a minimal medium containing MWF or MWF components as the sole carbon source (see van der Gast Env. Micro (2004) 6(3) 254-263). Flasks are incubated at about 100 rpm in a shaking incubator for about 16 hours. Cultured isolates are identified by DNA sequencing to exclude pathogens from consideration as inocula. Single species or consortia of microorganisms can be used to inoculate bioreactors. The matrix containing mature biofilm is removed from an operating bioreactor and transferred to the reactor that is to be inoculated. A volume of mature matrix biofilm that is approximately 10% of the volume of the reactor to be inoculated is used (*i.e.* a 500 ml tube of solid support matrix in a 5 litre bioreactor, 1000 litres into 10000 litre site reactor). The remaining volume of the reactor is occupied by clean (non-biofilmed) matrix. The reactor is then filled with diluted MWF waste and aeration is commenced immediately to avoid anaerobic action and hydrogen sulphide production. The COD level of the spent MWF is monitored measured. The non-biofilmed matrix is colonised and covered with biofilm over a period of days or weeks depending on the waste stream.

### Bioreactor commissioning

The bioreactor is commissioned at a temperature of about 18-20°C although temperatures down to 12°C can be used. Once mature, the bioreactors are able to tolerate about +1 to +35°C. COD reduction is negligible at 1°C, but operation can be sustained at a high level at 30°C for several days. Reactors are aerated using air from a compressor and injecting it into pipes at the bottom of the bioreactor to distribute it. The action of the bubbles rising provides agitation at the bioreactor surface but is not so violent as to dislodge attached biofilm. In practice this gives an air flow of about 250-300 litres per minute per 5000 litres of reactor volume. Dissolved oxygen levels are typically about 10 mg/L at the start of the commissioning process due to the low cell density. At maturity, measurable dissolved oxygen is typically less than 1 mg/L due to microorganism utilisation of free oxygen to oxidise the oils and other MWF components.

### Bioreactor feed

During the early commissioning period the liquid inoculum or biofilm inoculated bioreactor is fed dilute MWF waste of between about 5000 to about 10000 mg/L COD to minimise the toxic shock imposed by toxic components of the waste stream - such as biocides. At laboratory scale reactors are sometimes supplemented with tryptic soya broth. This is not practical or cost effective at full scale. At >1000 litre scale, an extremely low level of supplement is used (1-10 µl per litre of reactor volume) with a seaweed based trace element solution.

### Example 2 - A comparison of the performance of the R4T consortium versus the Microcycle consortium on Hocut® 795B.

A waste stream of Hocut® 795B is treated in solid-matrix inoculated bioreactors that are inoculated with either R4T or Microcycle. Hocut® 795B is a semi-synthetic MWF.

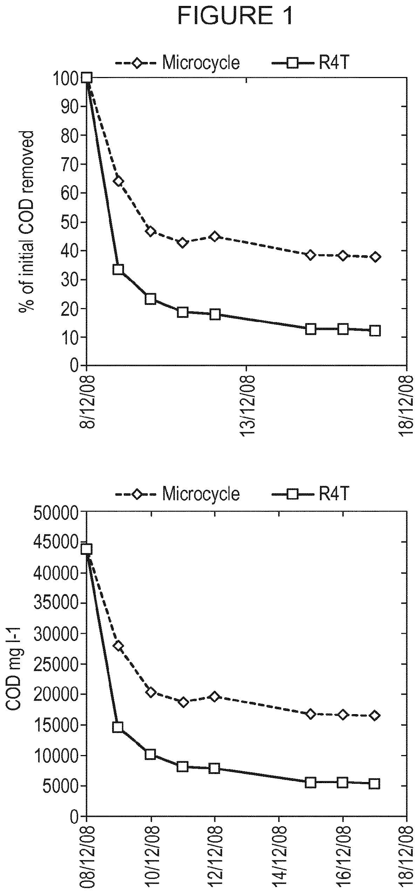

The starting COD of Hocut® 795B is about 45,000 mg/L and the reduction in COD is monitored over a period of 10 days. The results of this experiment are presented in Figure 1. The rate and extent of degradation is significantly greater for the R4T consortium than for the Microcycle consortium. The bottom hand figure shows the raw COD data. The upper figure shows the data as % COD reduction. After 10 days, the COD for the Microcycle consortium is reduced to about 17,000 mg/L, whereas for the R4T consortium, this is reduced to about 5,000 mg/L. In addition, the reduction in COD occurs more quickly for the R4T consortium as compared to the Microcycle consortium.

It is concluded that the R4T consortium is more effective at reducing the COD of semi-synthetic MWF as compared to the Microcycle consortium.

### Example 3 - A further comparison of the performance of the R4T consortium versus the Microcycle consortium on Hocut® 795B.

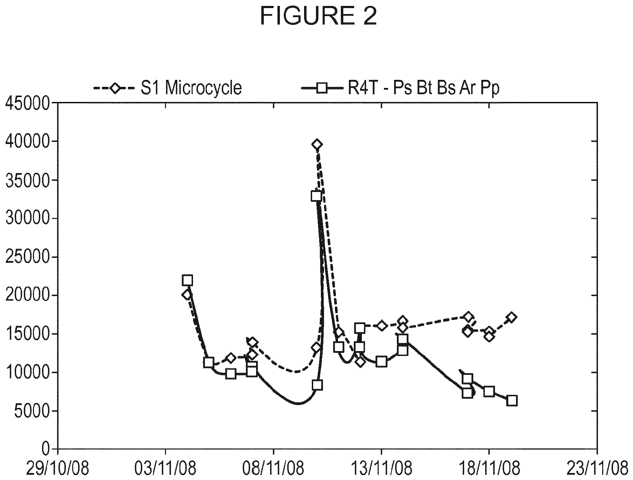

A waste stream of Hocut® 795B is treated in solid-matrix inoculated bioreactors that are inoculated with either R4T or Microcycle. Hocut® 795B is a semi-synthetic MWF.
Two experiments are carried out over a period of 7 days and over a period of 10 days. The results are presented in Figure 2.

In the first experiment (shown on the left hand side of Figure 2), the R4T consortium decreases the COD by 13,620 mg/L compared to only 6,800 mg/L by the Microcycle consortium, an increased efficiency of 50%. The COD level that is reached with the R4T consortium at the end of the trial is much lower than the COD level reached with the Microcycle consortium. The COD also reaches a lower level in a shorter period of time with the R4T consortium.

In the second experiment (shown on the right hand side of Figure 2), a 15% improvement was evident. The COD level that is reached with the R4T consortium at the end of the trial is much lower than the COD level reached with the Microcycle consortium. The COD also reaches a lower level in a shorter period of time with the R4T consortium.

The trend for the two experiments shows the performance of the R4T consortium improving. In contrast the Microcycle consortium has stabilized at a lower performance. The divergence between the performances of the two consortia would be expected to increase as the R4T consortium matures and becomes more robust.

It is concluded that the R4T consortium is more effective at reducing the COD of semi-synthetic MWF as compared to the Microcycle consortium.

### Example 4 - A further comparison of the performance of the R4T consortium versus the Microcycle consortium on a 10% MWF emulsion.

A waste stream of a 10% MWF emulsion (that is not Hocut® 795B) is treated in solid-matrix inoculated bioreactors with either R4T or Microcycle.

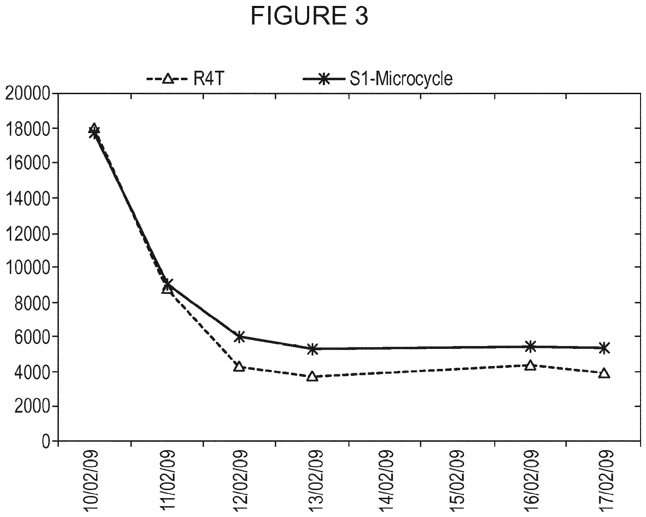

The results of this experiment are presented in Figure 3. The R4T consortium reduced the COD more effectively than Microcycle and also reached a lower COD in a shorter processing time.

It is concluded that the R4T consortium is more effective at reducing the COD of the 10% MWF emulsion as compared to the Microcycle consortium.

### Example 5 - Bioreactor performance assessment on industrial scale

An industrial scale bioreactor was set up at an automotive plant comprising the consortium of microorganisms described herein to demonstrate the reduction in COD achievable starting with effluent from the automotive plant.

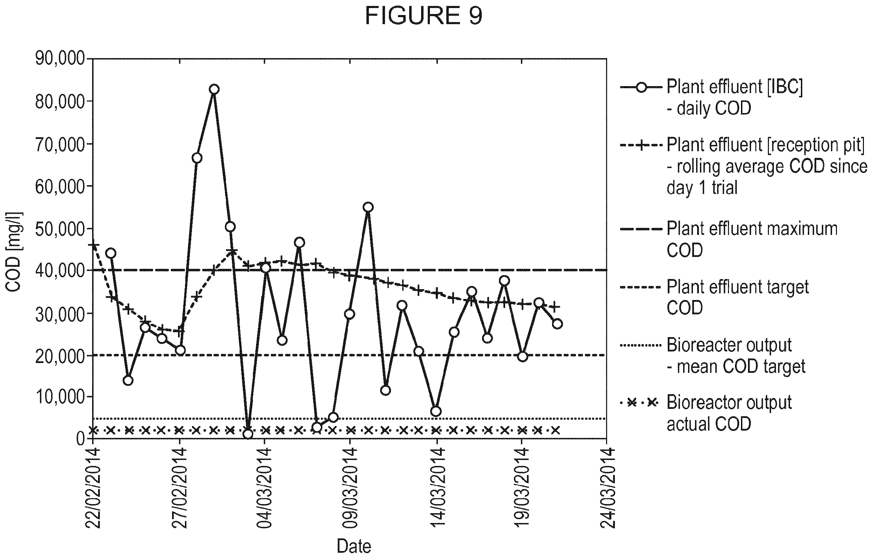

Figure 9 shows the performance of the bioreactor in detail during a trial period from 22 February to 24 March 2014. The actual effluent COD in waste fluids from the automotive plant, and therefore the inputs for the bioreactor varied greatly and went up to almost 85,000 mg/L. This is in contrast to the intention for a maximum plant effluent COD to be approximately 40,000 mg/L as shown by the horizontal dashed line. Current plant effluent target COD is 20,000 mg/L shown by the upper dotted horizontal line and the bioreactor target output COD was expected to be about 5,000 mg/L shown by the lower dotted horizontal line.

The bioreactor achieved much better than target COD outputs, as shown by the •x•x• line, of about 2,000 mg/L. This confirms the novel consortium of microoragnisms is highly effective and can reduce COD levels of effluent with varying high COD.

## Claims

1. A consortium of micro-organisms comprising, consisting or consisting essentially of *Rhizobium radiobacter* NCIMB 42280, *Bacillus subtilis* NCIMB 42282, *Bacillus thuringiensis* NCIMB 42283, *Pseudomonas putida* NCIMB 42441, and *Pseudomonas stutzeri* NCIMB 42281.

2. A biofilm comprising the consortium of micro-organisms according to claim 1, wherein the biofilm is colonised on a solid support.

3. A bioreactor for reducing the chemical oxygen demand (COD) of spent metal working fluids (MWFs), the bioreactor comprising the biofilm of claim 2, wherein the solid support is fixed to the bioreactor, optionally wherein at least a portion of the solid support is removable from the bioreactor, and optionally wherein the bioreactor further comprises spent MWF.

4. The bioreactor according to claim 3 further comprising:
(i) a second solid support matrix, wherein said second solid support matrix is not or is not substantially colonised by a biofilm of microorganisms; and
(ii) optionally, diluted spent MWF.

5. A method for reducing the COD of spent MWF, comprising contacting the MWF with the consortium according to claim 1, or the biofilm according to claim 2, or the bioreactor according to claim 3 or 4.

6. The method according to claim 5, comprising the steps of:
(a) providing the biofilm of claim 2 on a solid support matrix in a first bioreactor;
(b) transferring at least a portion of the solid support matrix comprising the biofilm of microorganisms from the first bioreactor into a second bioreactor; and
(c) incubating the microorganisms in the second bioreactor to reduce the COD of the spent MWF contained therein.

7. *Rhizobium radiobacter* NCIMB 42280.

8. *Bacillus subtilis* NCIMB 42282.

9. *Bacillus thuringiensis* NCIMB 42283.

10. *Pseudomonas putida* NCIMB 42441.

11. *Pseudomonas stutzeri* NCIMB 42281.

## Patentansprüche

1. Mikroorganismengemeinschaft, umfassend, bestehend aus oder im Wesentlichen bestehend aus *Rhizobium radiobacter* NCIMB 42280, *Bacillus subtilis* NCIMB 42282, *Bacillus thuringiensis* NCIMB 42283, *Pseudomonas putida* NCIMB 42441 und *Pseudomonas* stutzeri NCIMB 42281.

2. Biofilm, umfassend die Mikroorganismengemeinschaft gemäß Anspruch 1, wobei der Biofilm auf einem festen Träger besiedelt ist.

3. Bioreaktor um den chemischen Sauerstoffbedarf (CSB) von verbrauchten Metallbearbeitungsflüssigkeiten (MBF) zu verringern, wobei der Bioreaktor den Biofilm gemäß Anspruch 2 umfasst, wobei der feste Träger am Bioreaktor befestigt ist, wobei mindestens ein Teil des festen Trägers gegebenenfalls vom Bioreaktor entfernt werden kann und wobei der Bioreaktor ferner gegebenenfalls verbrauchte Metallbearbeitungsflüssigkeiten umfasst.

4. Bioreaktor gemäß Anspruch 3, ferner umfassend:
(i) eine zweite feste Trägermatrix, wobei die zweite feste Trägermatrix nicht oder nicht wesentlich mit einem Mikroorganismen-Biofilm besiedelt ist; und
(ii) gegebenenfalls verdünnte verbrauchte MBF.

5. Verfahren, um den CSB von verbrauchten MBF zu reduzieren, umfassend die MBF mit der Mikroorganismengemeinschaft gemäß Anspruch 1 oder dem Biofilm gemäß Anspruch 2 oder dem Bioreaktor gemäß Anspruch 3 oder 4 in Kontakt bringen.

6. Verfahren gemäß Anspruch 5, umfassend folgende Schritte:
(a) den Biofilm gemäß Anspruch 2 auf einer festen Trägermatrix in einem ersten Bioreaktor bereitstellen;
(b) die den Mikroorganismen-Biofilm umfassende feste Trägermatrix zumindest teilweise aus dem ersten Bioreaktor in einen zweiten Bioreaktor übertragen; und
(c) die Mikroorganismen im zweiten Bioreaktor inkubieren, um den CSB der darin enthaltenen verbrauchten MBF zu reduzieren; und

7. *Rhizobium radiobacter* NCIMB 42280.

8. *Bacillus subtilis* NCIMB 42282.

9. *Bacillus thuringiensis* NCIMB 42283.

10. *Pseudomonas putida* NCIMB 42441.

11. *Pseudomonas stutzeri* NCIMB 42281.

## Revendications

1. Un consortium de micro-organismes comprenant, constitué ou essentiellement constitué de *Rhizobium radiobacter* NCIMB 42280, *Bacillus subtilis* NCIMB 42282, *Bacillus thuringiensis* NCIMB 42283, *Pseudomonas putida* NCIMB 42441, et de *Pseudomonas stutzeri* NCIMB 42281.

2. Un biofilm comprenant le consortium de micro-organismes selon la revendication 1, dans lequel le biofilm est colonisé sur un support solide.

3. Un bioréacteur pour réduire la demande chimique en oxygène (DCO) des fluides pour le travail des métaux usés (FTM), le bioréacteur comprenant le biofilm selon la revendication 2, dans lequel le support solide est fixé au bioréacteur, éventuellement dans lequel au moins une partie du support solide peut être retirée du bioréacteur, et éventuellement dans lequel le bioréacteur comprend en outre des FTM usés.

4. Le bioréacteur selon la revendication 3 comprenant en outre :
(i) une deuxième matrice de support solide, dans lequel ladite deuxième matrice de support solide n'est pas ou n'est pas sensiblement colonisée par un biofilm de micro-organismes ; et
(ii) éventuellement, des FTM usés dilués.

5. Un procédé pour réduire la DCO de FTM usés, comprenant la mise en contact des FTM avec le consortium selon la revendication 1, ou le biofilm selon la revendication 2, ou le bioréacteur selon la revendication 3 ou 4.

6. Le procédé selon la revendication 5, comprenant les étapes consistant à :
(a) fournir le biofilm selon la revendication 2 sur une matrice de support solide dans un premier bioréacteur ;
(b) transférer au moins une partie de la matrice de support solide comprenant le biofilm de micro-organismes à partir du premier bioréacteur dans un deuxième bioréacteur ; et
(c) incuber les micro-organismes dans le deuxième bioréacteur pour réduire la DCO des FTM usés contenus dans celui-ci.

7. *Rhizobium radiobacter* NCIMB 42280.

8. *Bacillus subtilis* NCIMB 42282.

9. *Bacillus thuringiensis* NCIMB 42283.

10. *Pseudomonas putida* NCIMB 42441.

11. *Pseudomonas stutzeri* NCIMB 42281.
